# EUROPEAN PATENT SPECIFICATION

(11) **EP 2 459 133 B1**
(45) Date of publication and mention of the grant of the patent: **21.01.2015**
(21) Application number: 10739670.7
(22) Date of filing: 28.07.2010
(51) Int. Cl.: A61F 9/00

(54) **DISPENSER WITH SLIDING SEAL, TREATMENT DEVICE AND RELATED METHODS**
SPENDER MIT GLEITDICHTUNG, BEHANDLUNGSVORRICHTUNG UND ZUGEHÖRIGE VERFAHREN
DISTRIBUTEUR AVEC JOINT COULISSANT, DISPOSITIF DE TRAITEMENT ET PROCÉDÉS AFFÉRENTS

(30) Priority: 28.07.2009 GB 0913127
(43) Date of publication of application: 06.06.2012
(73) Proprietor: PA Knowledge Limited, London SW1W 9SR (GB)
(72) Inventor: WHITAKER, David, Jonathan, Townley, Hertfordshire SG8 6DP (GB); RUSSELL, David, Morrison, Hertfordshire SG8 6DP (GB); KEMP, Thomas, Mark, Ashwell SG7 5NW (GB); NELSON, Craig, Harvey, Hertfordshire SG8 6DP (GB)
(74) Representative: Forsythe, Dominic
(86) International application number: PCT/GB2010/001436
(87) International publication number: WO 2011/012856

(56) References cited:
- WO-A1-01/08732
- WO-A1-2009/073482
- US-A1- 2004 220 537

## Description

The present invention relates to devices and methods for dispensing metered doses of a fluid for use in treatment of the human or animal body, in particular for treatment of the eye, or for systemic treatment via the eye.

Various devices are available in the prior art for providing doses of ocular fluid.

For example, dropper bottles containing a large number of doses of an ocular fluid are in widespread use. A dose from such a device is typically applied by positioning the bottle above the eye and squeezing or otherwise manipulating the bottle to cause a single drop to fall from the opening of the bottle into the eye. This operation can be facilitated by controlling the surface tension of the fluid and the material and/or size and shape of the bottle opening. Nevertheless, the action can be difficult to carry out, particularly for fragile patient groups, as it requires sustained elevation of the bottle and a significant degree of force and control.

Typically, once the bottle has been opened, the fluid is no longer sterile. This means that a preservative may need to be added, which increases cost and a significant number of patients are allergic to such preservatives. The bottle may also need to be kept chilled after opening, which may not be convenient. More generally, the loss of sterility may reduce the shelf-life and/or safety of the product.

In practice, the size of drops dispensed by dropper bottles varies substantially, which can lead to uncertainty about the dose being applied to the patient. More generally, dropper bottles tend to be effective only for relatively large drops, i.e. drops containing more fluid than can actually be sustained on the eye. For example, dropper bottles may dispense drops of between 50µl and 100µl, whereas the eye can only sustain about 20µl. This results in unpleasant "run-off" during application and waste of the product being dispensed.

Blow-fill-sealed single ampoules can be used as an alternative to the dropper bottle. As the ampoules are intended for single use and are only opened for the first time immediately before use, sterility is reliably maintained without any added preservatives. However, the method of application is very similar to that of the dropper bottle and the same disadvantages are encountered. Furthermore, ampoules have a twist-off portion which must be removed to access the dose, the process of which can create irregularities at the dropper tip which can further reduce the accuracy of droplet size and form a dangerous or uncomfortable surface for bringing into contact with or close to the eye. In particular, the delivery action is difficult to carry out and the drop size tends to be larger than the eye can sustain, leading to unpleasant run-off and wastage. Furthermore, manufacturing and filling one ampoule for each use is expensive in comparison to the dropper bottle, which can house many doses of the fluid.

US 2004/0220537 A1 discloses an ocular treatment device comprising a unit container for a treatment fluid having a sealed enclosure of which one wall section thereof is formed with at least one opening. The enclosure is pressurisable to discharge its contents through the opening or openings, which is or are of sufficient diameter to enable the generation of a jet and/or discrete droplets of treatment fluid discharged therefrom. The one wall section is typically a flat section of the enclosure wall, and the enclosures typically a blister pack, with the wall section at a planar base of the blister. However, the one wall section may be dome-shaped and formed with at least one opening in the top region of the dome. Containers of the disclosure may be provided in packages, for example in strip form or in planar arrays. Dispensing devices are described for discharging their content in treatment.

It is an object of the present invention to at least partially overcome some of the problems with the prior art mentioned above.

According to an aspect of the invention, there is provided a treatment device, comprising: a fluid dispenser comprising: a sealable reservoir for storing a fluid to be dispensed; a transfer body for carrying a predetermined volume of said fluid from said reservoir to a dispensing point outside of said reservoir; and a sliding seal for maintaining said reservoir in a sealed state while a portion of said transfer body carrying said predetermined volume of fluid and said predetermined volume of fluid are moved from a position inside of said reservoir to said dispensing point outside of said reservoir; and an applicator for applying a predetermined volume of fluid present at said dispensing point to a part of the human or animal body, wherein said applicator comprises means for dispersing said predetermined volume of fluid in a jet of gas directed towards said part of the human or animal body.

The sealed state of the reservoir is at least such that fluid is substantially contained within the reservoir and cannot pass through to the outside of the reservoir in any significant quantity except by movement of the transfer body through the sliding seal. Preferably, the sealed state is such as to inhibit movement of potential contaminants from the outside of the reservoir to the inside of the reservoir. More preferably, the sealed state is such that the sterility of the reservoir is maintained to a sufficiently great extent that preservatives are not required within the fluid of the reservoir.

These arrangements provide cost and convenience advantages similar to those associated with bulk-filling systems but without many of the associated problems of the prior art. For example, smaller volumes than are possible using dropper bottles and the like are possible, thus avoiding run-off and waste. The small volumes and mechanical presentation of the fluid at the dispensing point mean that convenient application mechanisms are available, such as those using jets of gas to deliver the fluid onto the region to be treated. The dosage can also be controlled more accurately and with greater reproducibility. Where the sealed state is such as to maintain sterility, the use of preservatives can be avoided.

The predetermined volume of fluid may be carried by being trapped within a volume defined by the transfer body and which moves with the transfer body, in the same direction as the transfer body, as the transfer body is driven through the sliding seal.

The degree of sealing (level of "sealed state") achieved by the sliding seal may be adapted according to the particular application for which the dispenser is intended.

The transfer body may be formed so to define a cavity capable of containing said predetermined volume of fluid, said transfer body being arranged such that, in use, movement of a portion of said transfer body defining said cavity from a position inside of said reservoir to a position outside of said reservoir causes said predetermined volume of fluid to be transferred from said reservoir to said dispensing point outside of said reservoir.

Alternatively, the cavities may be formed as spaces between adjacent segments of a segmented transfer body.

Furthermore, the fluid dispenser may be configured such that a plurality of cavities can simultaneously occupy the dispensing position (i.e. for fluid to be dispensed from a plurality of cavities simultaneously or sequentially) in order to deliver the volume of fluid required. This may be advantageous from the point of view of maximizing the contact area between the inner surfaces of the cavities and the fluid to help ensure that the fluid remains in the cavities (due to surface tension effects) from when the transfer body leaves the sliding seal until the delivery process takes place (e.g. a jet of gas blows the liquid out of the cavities).

The fluid dispenser may be configured such that a portion of the transfer body located inside of said reservoir can adopt a compact configuration relative to the configuration that the transfer body adopts as it is forced through the sliding seal. The compact configuration may be a coiled configuration, for example, or a configuration in which segments of the transfer body approach each other axially due to collapsing or bending of flexible connecting members between the segments or both. The compact arrangement may be compact in three dimensions, or in only one or two dimensions, according to a desired aspect ratio of the device in which it is to be used for example.

The cavities may each have an opening into the reservoir that may be elongated so as to be narrower in a direction parallel to a direction of insertion of said cavity through said sliding seal. This reduces the length over which the sliding seal needs to be effective, thus reducing the cost and/or improving the performance of the sliding seal and/or compactness of the device.

The reservoir may be configured to reduce in volume as the volume of fluid stored in said reservoir is reduced. This reduces wastage of the product to be dispensed and avoids a reduction of pressure in the reservoir.

The interior surface of the cavities may be arranged to be more hydrophilic than other surfaces of said transfer body in the region of said cavity. This arrangement encourages fluid to be held within the cavities and reduces the risk and/or extent of leak of fluid out onto the surfaces surrounding the openings to the cavities, thus helping to improve dose accuracy.

The interior surface of the cavity may be formed such that a contact angle with water and/or with the fluid to be dispensed is less than 45 degrees, more preferably less than 30 degrees, and more preferably still less than 15 degrees.

A portion of the surface of the transfer body surrounding an opening of the cavity may be formed such that a contact angle with water and/or with the fluid to be dispensed is greater than 45 degrees, more preferably greater than 75 degrees, more preferably greater than 90 degrees and more preferably still greater than 105 degrees.

The fluid may be for applying a treatment via the eye (i.e. for treating the eye itself or for treating another part of the body via the eye).

The fluid may comprise one of the following: a liquid, a suspension, a solution, an emulsion, a slurry, a gel, a semi-solid and a gas.

According to an alternative aspect of the invention, there is provided a method for dispensing a fluid, comprising: sealing a fluid to be dispensed in a reservoir; using a transfer body to carry a predetermined volume of fluid from said reservoir to a dispensing point outside of said reservoir; using a sliding seal to maintain said reservoir in a sealed state while a portion of said transfer body carrying said predetermined volume of fluid and said predetermined volume of fluid are moved from a position inside of said reservoir to said dispensing point outside of said reservoir; generating a jet of gas directed towards a part of a human or animal body to be treated; and dispersing the predetermined volume of fluid present at said dispensing point in said jet of gas.

The treatment device may be configured such that one or a plurality of cavities can be selectively presented at the dispensing point and the fluid contained therein simultaneously applied, the volume of fluid thus applied depending on the number and nature of the cavities selectively presented at the dispensing point. In this way, a user can vary the dose to be applied easily and without having to use the device a plurality of times, for example by repeatedly applying a standard invariable dose the required plurality of times.

The invention will be more clearly understood from the following description, given by way of example only, with reference to the accompanying drawings, in which:
Figure 1a shows a dispenser comprising an elongate, "rod-like" transfer body;
Figure 1b is a top view of a transfer body according to Figure 1a, showing an example cavity opening profile;
Figure 1c is a top view of a transfer body of Figure 1a, showing an alternative (elongated) cavity opening profile;
Figure 2a shows a tape-like embodiment of the transfer body;
Figure 2b shows the transfer body of Figure 2a in a coiled configuration;
Figure 3 a shows a segmented embodiment of the transfer body;
Figure 3b shows the transfer body of Figure 3a in a coiled configuration;
Figure 3c shows a transfer body of the type shown in Figures 3a and 3b, with flexible filler members for ensuring a longitudinally constant cross-sectional profile;
Figure 3d shows a dispenser comprising a transfer body of an alternative segmented embodiment;
Figure 3e shows a segment of a transfer body according to an alternative segmented embodiment;
Figure 3f shows a dispenser comprising a transfer body having a string of segments of the type shown in Figure 3e;
Figure 3g shows a dispenser in which a segment protruding from the sliding seal can be twisted to change the separation between that segment and an immediately preceding segment to vary the dose to be presented at the dispensing point;
Figure 4 shows a treatment device comprising a dispenser having a tape-like transfer body wound onto reels;
Figure 5a shows a dispenser comprising transfer bodies consisting of a vertical stack of segments and means for pushing the segments through a sliding seal;
Figure 5b is an end view of the transfer bodies of Figure 5a;
Figure 5c is a side view of an isolated transfer body of the type used in the dispenser of Figure 5a;
Figure 5d is a top view of the stack of transfer bodies shown from the side in Figure 5a with vertical alignment rods acting to align and ensure the correct orientation of the transfer bodies prior to expulsion through the sliding seal;
Figure 6a shows a dispenser having a rod-like transfer body and a reservoir delimited by an elastic sleeve;
Figure 6b shows the dispenser of Figure 6a after a number of doses have been dispensed, with the elastic sleeve contracted to reduce the size of the reservoir;
Figure 6c shows the dispenser of Figures 6a and 6b in a coiled configuration;
Figure 7 shows an alternative dispenser in which only a subset of the cavities (less than all of the cavities) are filled with fluid prior to expulsion through the sliding seal; and
Figure 8 illustrates the definition of contact angle.

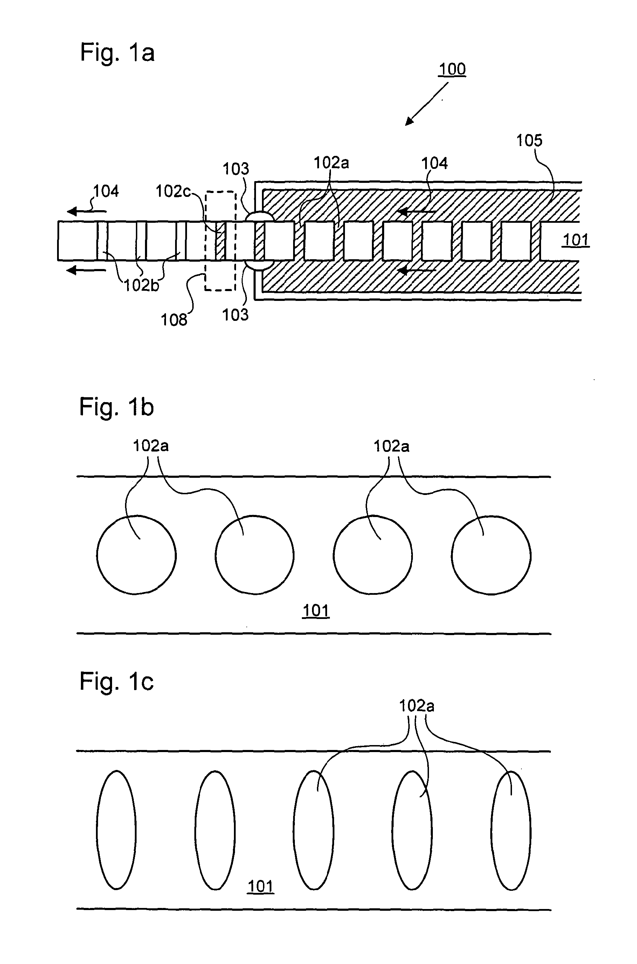

Figure 1a shows a side view of a dispenser 100 according to an embodiment of the invention. A fluid to be dispensed, for example an ocular fluid for eventual application to the eye, is stored in the reservoir 105. The reservoir 105 is filled in such a way that the fluid in the reservoir 105 is initially sterile, for example in a clean environment. Alternatively or additionally, the contents of the reservoir 105 can be sterilized after filling, by irradiation for example. Preferably, no preservatives are added to the fluid.

A transfer body 101 is provided for transferring fixed doses of the fluid from the reservoir 105 to a dispensing point 108 outside of the reservoir through a sliding seal 103. According to the embodiment shown, the transfer body 101 has an elongated "rod-like" form. In this embodiment, the cross-sectional shape of the rod is typically constant, at least for the portion of the rod that is to seal against the sliding seal 103. Preferably, the rod has a substantially circular cross-section. More generally, the outer cross-sectional profile of the transfer body 101 at axial positions of maximum radial size should preferably be constant, and the axial distance between the axial positions of maximum radial size should be less than the axial width of the sliding seal. Such arrangements facilitate efficient operation of the sliding seal 103.

In the case of an axially non-continuous cross-section (i.e. a cross-section varying as a function of axial position along the transfer body 101), in order to ensure seal integrity during transfer, the transfer body 101 may make contact with the sliding seal 103 at more than one discrete point. For example, at least at certain times, the sliding seal 103 may simultaneously make contact with two adjacent regions of maximum radial size of an axially non-continuous transfer body.

In use, the transfer body 101 will be positioned so as to have a portion projecting into the reservoir 105, preferably submerged within the fluid contained therein, and a portion projecting outside of the reservoir 105 to and/or through the dispensing point 108.

Cavities 102a/102b/102c are formed within the body 101 and have openings to the exterior of the transfer body 101 such that, when the cavities are located in the reservoir, fluid can enter the cavities, preferably so as to completely fill them.

Fluid is transferred to the dispensing point 108 outside of the reservoir 105 by moving the transfer body 101 longitudinally (arrows 104) by pushing, pulling, twisting, turning or combinations thereof, so as to shift a cavity 102a/102b/102c from a position inside the reservoir 105 through the sliding seal 103 to the dispensing point 108. In the arrangement of Figure 1a, cavities 102a are inside the reservoir 105 and cavities 102b and 102c are outside of the reservoir 105; cavity 102c still contains fluid to be dispensed and is located at the dispensing point 108 ready for delivery, while the cavities 102b have passed the dispensing point 108 and no longer contain any fluid (because it has been dispensed).

The quantity of fluid presented in this way at the dispensing point 108 depends almost entirely on the volume of the cavities 102a/102b/102c and is accurately and reproducibly controllable. In addition, in comparison to prior art approaches such as the dropper bottle and blow-fill-sealed ampoules, smaller volumes of fluid can be dispensed more easily. For example, where the dispenser is to be used for dispensing ocular fluid, the dose volume may be controlled so as to be sufficiently small that substantially all of the dose can be sustained on the eye. Waste and unpleasant "run-off" (leakage or overflow of fluid from the eye) can thus be minimised or avoided. Preferably, the dispensed volume may be lower than 25 microlitres, lower than 20 microlitres, lower than 15 microlitres, lower than 10 microlitres, or lower than 5 microlitres.

In the arrangement shown, only a single filled cavity 102c is presented at the dispensing point 108. However, the fluid dispenser 100 may be configured such that a plurality of filled cavities can occupy the dispensing position 108 and fluid can be dispensed simultaneously from all of these cavities. This approach may be helpful from the point of view of maximizing the contact area between the inner surfaces of the cavities 102 and the fluid to help ensure that the fluid is held effectively in the cavities 102 by surface tension from when the transfer body 101 leaves the sliding seal 103 until a delivery process (comprising, for example, a jet of gas blowing liquid out of the cavities) is about to take place. Alternatively or additionally, the fluid dispenser 100 may be configured so that the number and/or nature (e.g. volume) of the cavities 102c presented at the dispensing point 108 (filled with fluid ready for delivery) can be selectively varied by a user in order to vary the dose applied. For example, in the case where all of cavities 102 are the same volume and arranged sequentially, the fluid dispenser 100 may be configured such that a single operation of a transfer body actuator (not shown) brings one filled cavity 102c to the dispensing point 108, while two consecutive operations of the transfer body actuator brings two filled cavities 102c to the dispensing point 108. In this way, initiation of the delivery mechanism (e.g. jet of gas) after a single operation of the transfer body actuator allows a single dose to be delivered (which may be suitable for a child, for example), whereas initiation of the delivery mechanism after two operations of the actuator allows a double dose to be applied (which may be suitable for an adult, for example). A user can thus apply different doses without having to use the device a plurality of times (for example by repeatedly applying a standard dose the required number of times).

The sliding seal 103 maintains a seal against the transfer body 101 while the transfer body 101 moves ("slides") through it. The sliding seal 103 is preferably arranged so that for all positions of the transfer body 101 relative to the reservoir 105, there is never any path for a contaminant to reach the fluid in the reservoir 105 from the outside environment and thereby compromise sterility.

For example, the sliding seal 103 may be arranged so as to be longitudinally wider (axially longer) than the openings of the cavities 102a/102b/102c, such that fluid in the cavities 102a/102b/102c is never exposed simultaneously to the reservoir 105 and the region outside of the reservoir 105.

For example, the sliding seal 103 may consist of a suitably dimensioned "o-ring" and groove, a gland seal, two appropriately spaced lip seals, or biased o-rings. Alternatively or additionally, the sliding seal 103 may be implemented by arranging for the transfer body 101 to be compliant and to cooperate with a suitably dimensioned rigid sealing body. The sealing may be based on achieving an interference fit between the transfer body 101 and sliding seal 103.

The cavities may have two or more openings into the fluid. For example, the cavities 102a/102b/102c may be formed by removing material from a solid transfer body 101 (e.g. by stamping), or by inherently forming cavities (e.g. injection moulding). The cavities may be arranged so as to have openings facing each other on opposite sides of the transfer body 101, for example. The volume of the cavities formed in this way can be precisely controlled at relatively low cost, because a variety of industrial means are available. The risk of bubbles in the cavities, which could compromise dose accuracy, may be reduced in cavities having two or more openings in comparison to cavities having only a single opening. In any event, the risk of bubbles could be further reduced by arranging for the cavity to be filled in a vacuum environment or by otherwise ensuring that the reservoir is completely filled and no gas is present.

The profile of the cavities may be circular, elliptical, polygonic or otherwise shaped in a manner that facilitates filling, sealing or dosing. Figure 1b, which is a top view of a transfer body 101 in accordance with Figure 1a, shows cavities 102a having a circular.cross-section, which are particularly easy to manufacture accurately.

Additionally, again for facilitating particular filling, sealing or dosing methods, the cavities themselves may be tapered or have a stepped profile and/or may have one or more discrete openings in either side of the transfer body. The surface properties of the cavities and surrounding areas of the transfer body 101 may also be modified so as to assist with complete filling of the cavities. For example, the interior surfaces of the cavities may be arranged to have relatively hydrophilic properties (and/or relatively low contact angle with respect to the fluid to be dispensed) and/or the surfaces of the areas of the transfer body 101 surrounding the holes may be arranged to have relatively hydrophobic properties (and/or relatively high contact angle with respect to the fluid to be dispensed).

In order to facilitate operation of the sliding seal 103, the cavities may be formed so as to have openings which are elongate in a direction perpendicular to the longitudinal direction of the transfer body 101 (i.e. narrower in a direction parallel to the insertion direction). Figure 1c, which is a top view of a transfer body 101 in accordance with Figure 1a, illustrates such an arrangement. As mentioned above, in order to ensure maintenance of the sterility of the reservoir 105, the sliding seal 103 should maintain a seal against the transfer body 101 over a longitudinal distance at least as great as the longitudinal width of the openings. Arranging for the openings to be elongate in a direction perpendicular to the longitudinal direction of the transfer body 101 effectively reduces the longitudinal width of the openings for a given cavity volume and therefore reduces the longitudinal distance over which the seal has to be maintained.

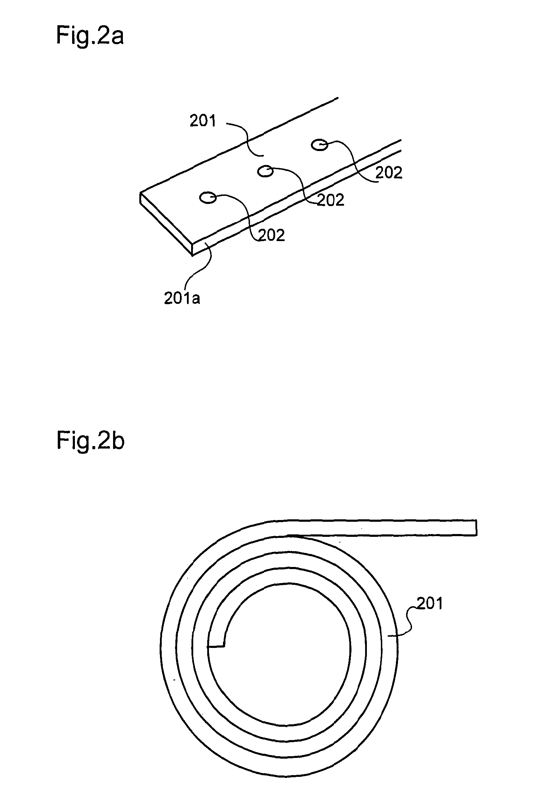

Figures 2a and 2b illustrate an alternative embodiment of the transfer body 201. Figure 2a is a perspective view of a straightened portion of the transfer body 201 and Figure 2b is a side view of the transfer body 201 in a coiled configuration. The transfer body 201 according to this embodiment is shaped so as to have a strip-like or tape-like form. In other words, the cross-sectional profile is elongated or relatively flat in a direction perpendicular to a longitudinal direction of the transfer body 201. The width is substantially greater than the height, for example, the ratio of width to height may be 100:1, 50:1, 10:1 or 5:1.

As in previous embodiments, the cross-sectional profile is preferably substantially constant at least for portions of the transfer body 201 that will come into contact with (be acting to seal against) the sliding seal 103 in use. The cavities 202 may be formed by drilling through the transfer body 201, by stamping or by moulding, for example. The sliding seal 103 may be configured to operate as a lip seal, for example. The lateral side edges 201 a of the transfer body may be shaped (e.g. tapered) to facilitate sealing.

The transfer body 201 may be configured so that it can be bent or otherwise shaped into a compact form within the reservoir. This enables the aspect ratio of the reservoir to be changed and may help to increase the proportion of the fluid in the reservoir that can be dispensed by the transfer body 201. For example, the transfer body 201 may be formed of a flexible material that can be wound into a coiled formation, such as that shown in Figure 2b.

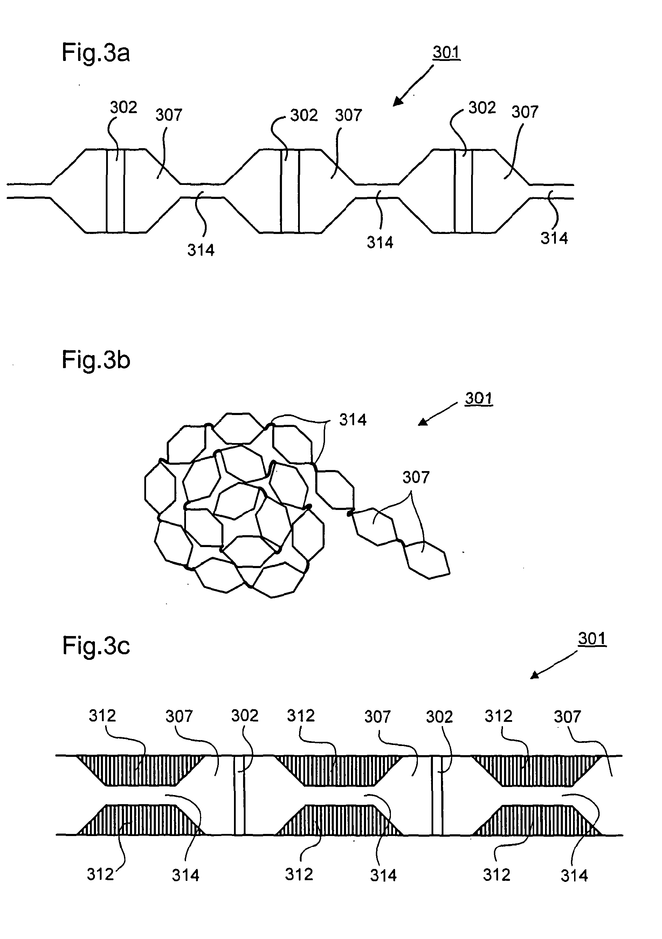

Figures 3a and 3b show a further alternative embodiment of the transfer body 301. Again, this embodiment can be shaped so as to adopt a compact configuration within the reservoir 105. Figure 3a is a side view of the embodiment in a straightened configuration and Figure 3b shows the same transfer body 301 in a coiled configuration.

The transfer body 301 according to this embodiment comprises regions 307 of greater thickness ("segments") within which are formed the cavities 302 (which may be cylindrical, for example) for containing the fluid to be dispensed. In the example shown, each of the segments 307 comprises a single cavity, but two or more cavities 302 could also be located within each one of the segments 307. The segments 307 are connected to each other by flexible connectors 314 (that is, portions having a different stiffness than the segments 307), which may comprise thinner regions of the same material as the rest of the transfer body 301 and/or may be formed from material different to the rest of the transfer body 301. The connectors 314 facilitate shaping of the transfer body 301 into a compact form. For example, the portion of the transfer body within the reservoir may adopt a coiled formation as shown schematically in Figure 3b. Alternatively, the cross-sectional profile of the transfer body 301 may be kept constant (so as to facilitate operation of the sliding seal) and the enhanced flexibility of the connectors 314, for the purposes of coiling, may be entirely achieved using a suitably flexible material (different from that used for the segments 307).

Figure 3c shows a further alternative arrangement in which a core of the transfer body has a form as shown in Figure 3a, comprising segments 307 within which are formed the cavities 302, and flexible connectors 314 for connecting together adjacent segments 307 in such a way as to allow the transfer body to adopt a compact configuration within the reservoir. According to the example shown, the segments 307 and the flexible connectors 314 are formed from the same material and the relative flexibility of the flexible connectors 314 is achieved by making these portions sufficiently thin. In order to facilitate operation of the sliding seal, the transfer body 301 according to this embodiment further comprises flexible filler members 312 (formed from material that is more flexible than that of the segments 307) which completely fill the gaps between adjacent segments 307 and ensure that the outer cross-sectional profile of the transfer body 301 is constant along its length, but which still allow bending of the flexible connectors. This structure could be manufactured efficiently using a two shot injection moulding process for example.

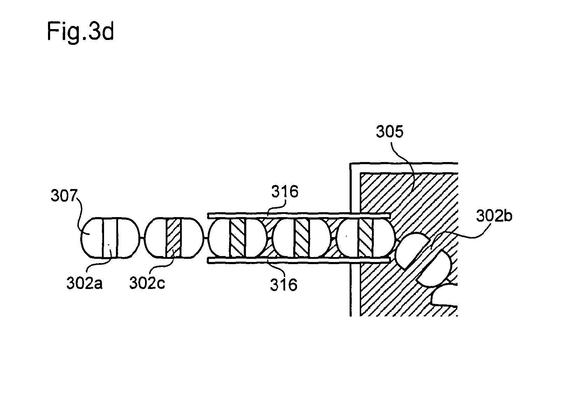

Figure 3d is a side view of an alternative embodiment of the type shown in Figures 3a to 3c. Here, segments 324 are again connected to each other via a non-rigid, flexible member 314, which allows the segments to adopt a compact configuration within the reservoir 305, thereby saving space. In contrast to the arrangements of Figures 3a to 3c, however, the flexible member 314 is here formed from a different material than that of the segments 307. Each of the segments 307 has rounded corners to assist with adoption of a compact configuration within the reservoir 305. In this embodiment, the compact configuration may be achieved through "coiling" (or any other deviation from a longitudinally aligned form that is more compact) or through an axial collapse of the flexible member 314 between segments, or a combination of both. As in the embodiment of Figures 3a to 3c, the segments 307 can be pulled through the sliding seal 316 by applying a pulling force to segments 307 that have already passed through the sliding seal 316. In the embodiment shown, the segments 307 have a circular cross-section, with a cylindrical vertical cavity 302a/b/c for containing the fluid to be dispensed.

As a further alternative embodiment (not shown), the transfer body may be formed from segments thread onto a continuous string-like flexible member in the manner of beads onto a necklace, but with the flexible member sealed within each of the segments. This arrangement allows very effective adoption of compact configurations with the reservoir, thus saving space. Cavities would be defined within each segment (like in the embodiment of Figure 3a or 3d, for example), but would generally need to be arranged so as to avoid the cavities through which the flexible member is to run (preferably, the flexible member would run through an axially centred cavity in each segment, for example). For example, in the case where the cavity for the flexible member is axially centred, the cavities for carrying the fluid to be dispensed may be arranged so as to be offset from an axial centreline of the segments.

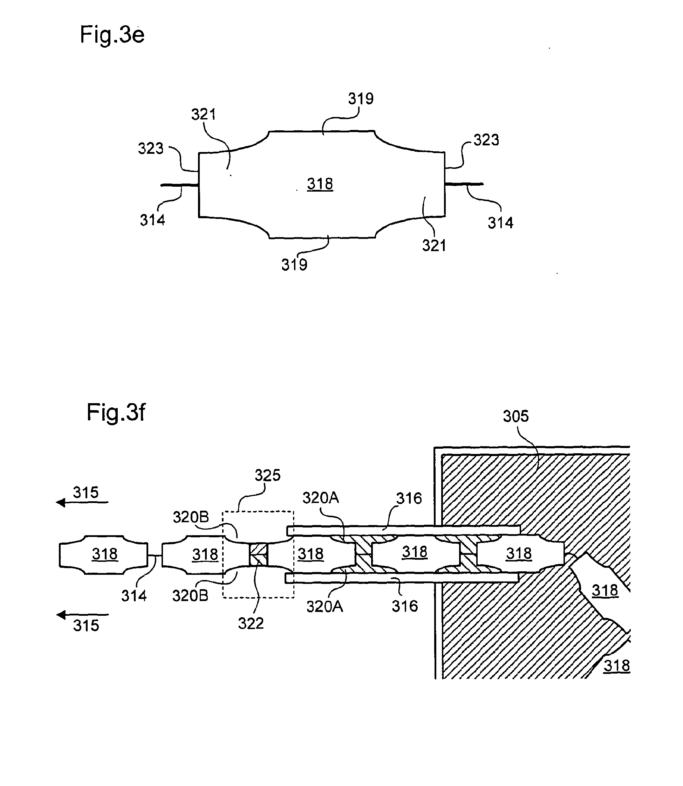

Figures 3e and 3f show a segmented transfer body 301 in which the individual segments 318 are formed so as to trap and convey fluid in two different volumes defined by the space between the segments 318 when the segments 318 are within the sliding seal 316: a dispensing volume 322 and a buffer volume 320A/B. The dispensing volume 322 is entirely surrounded by the buffer volume 320A/B, in the sense that all openings into the dispensing volume 322 open out into the buffer volume 320 A/B. Thus, when the buffer volume 320 A/B is completely full of fluid, the dispensing volume 322 is also full and all openings into the dispensing volume 322 are covered by the fluid within the buffer volume 320 A/B.

The buffer and dispensing volumes may be arranged so that when the surrounding segments have been driven through the sliding seal 316, fluid within the buffer volume 320A/B flows away under the action of gravity, while the fluid within the dispensing volume 322 is held in place by surface tension. This can be achieved by controlling the contact angles (see below for definition) of the surfaces of the segments 318 defining the buffer and dispensing volumes relative to the fluid to be dispensed, as well as by controlling the geometry of the buffer and dispensing volumes. In particular, the dispensing volume 322 should preferably be configured so that the energy associated with keeping the dispensing volume full of fluid to be dispensed is sufficiently low to provide an energy barrier against the fluid falling out under the influence of gravity or other forces that are expected to be encountered in normal use, while at the same time allowing the fluid to be dispensed effectively. In practice, this will mean forming the interior surfaces of the dispensing volume 322 (or coating them) so that they are relatively hydrophilic (and/or have a relatively low contact angle with respect to the fluid to be dispensed) and/or arranging for the geometry of the dispensing volume to be such as to present a relatively large contact area between the fluid and the hydrophilic interior surfaces. For example, the geometry of the dispensing volume may be arranged to be a narrow cylindrical tube with small longitudinal openings at each end or to have a substantially planar or flat form defined by opposed planar faces of neighbouring segments 318.

In contrast, the buffer volume 320A/B should preferably be arranged so that fluid flows away freely and quickly when the buffer volume 320 A/B moves clear of the sliding seal 316. This may be achieved by arranging for the interior surfaces of the buffer volume 320 A/B to be relatively hydrophobic (large contact angle) or at least substantially more hydrophobic than the interior surfaces of the dispensing volume 322. The shape of the buffer volume 320 A/B may also be chosen so that there is a continuous downhill route (i.e. a route that does not have any uphill portions) from all positions within the buffer volume, so that none of the fluid can be trapped in basins or other local "energy minima". Preferably, the size of the buffer volume 322 is made as small as is consistent with allowing it to carry out the functions described above, so as to minimize the loss of fluid. The depth of the buffer volume 322 may be substantially less than that shown in Figures 3e and 3f, for example, where features have been exaggerated for clarity.

In the specific example of Figures 3e and 3f, each of the segments 318 is cylindrically symmetric about a longitudinal axis of the sliding seal 316 and is attached to neighbouring segments 318 by a flexible member 314. The segments 318 each have a radially outermost portion 319 which will seal against the sliding seal 316 and radially innermost extensions 321 which extend axially towards nearest neighbour segments 318. The radially innermost extensions 321 end in opposing faces 323, which in the cylindrically symmetric example embodiment will be circular in form. When the segments 318 are axially aligned within the sliding seal 316, the opposing faces 323 will be parallel to each other, thus defining a disc-shaped space 322 between the opposing faces 323.

The flexible member 314 may have string-like properties, being for example relatively thin, strong in tension and non-rigid. This allows the segments 318 to move axially and to tilt relative to each other, which facilitates the adoption of compact configurations, for example coils, within the reservoir 305. The segments 318 may be driven through the sliding seal 316 by pulling on a portion of the transfer body that has already passed through the seal 316 (arrows 315). This action in combination with the friction between the segments 318 and the interior of the sliding seal 316 causes the flexible member 314 between segments 318 within the sliding seal 316 to become taut. The tautness of the flexible member 314 and the shape of the segments 318 (which encourages the segments 318 within the sliding seal 316 to be longitudinally aligned with each other) ensures that the geometry (and therefore volume) of the space between segments 318 within the sliding seal 316 is precisely defined.

The space between the segments 318 comprises two portions: the outer space 320 A/B, which has donut-like or toroid-like shape with a semi-circular cross-section, and the inter-segment space 322 between the opposing faces 323 of the segments 318 described above. The outer space 320 A/B is the "buffer volume" of this embodiment while the inter-segment space 322 is the "dispensing volume" of this embodiment.

Portions of fluid for dispensing are removed from the reservoir 305 by pulling the segments 318 through the sliding seal 316. A proportion of the fluid to be removed will be in the buffer volumes 320A and the rest in the dispensing volumes 322. When the dispensing and buffer volumes reach the dispensing point (dotted square 325), fluid flows out of the buffer volume 320B but remains in the dispensing volume 322 (due to surface tension), ready for dispensing.

The presence of the buffer volume 320 A/B helps to ensure that the dispensing volume 322 is properly filled prior to leaving the sliding seal 316. In addition, the fluid in the buffer volume 320 A/B acts as a barrier against interference with the fluid in the dispensing volume 322 from elements outside of the sliding seal 316, which might unpredictably alter the amount of fluid that is ultimately presented for dispensing. For example, in embodiments where the dispensing fluid is in direct contact with the sliding seal 316 (because there is no buffer volume to prevent it), there is a risk that some of the fluid will be left behind on the sliding seal 316 when the part of the transfer body carrying the fluid dose leaves the seal 316. For example, fluid could be dragged out of the transfer body while it is being pulled through the sliding seal 316 and/or fluid could stick to the front edge of the sliding seal 316 where the transfer body leaves the sliding seal. Such effects could be reduced by arranging for the interior and/or front edge of the sliding seal to be hydrophobic (and/or have a large contact angle with respect to the fluid to be dispensed), but the provision of a buffer volume 320 A/B mitigates the problem without requiring such measures. A small amount of the fluid in the buffer volume 320 A/B might be lost as the segments are driven through the sliding seal 316, but the dispensing volume 322, which is isolated from contact with the seal 316 by the fluid in the buffer volume 320 A/B, is not affected. The provision of the buffer volume 320 A/B thus potentially improves the accuracy and reproducibility with which fluid can be presented at the dispensing point 325.

The buffer fluid may be allowed to drain off directly to a waste sink, for example a waste receptacle, or directly to the environment (the choice will depend in general on the nature of the medicament and the quantity of buffer that is wasted per dose delivery). Alternatively or additionally, the buffer may be used to clean an element of the delivery system. For example, where the fluid is to be dispensed by blowing a jet of gas through the dispensing volume 322, the waste buffer fluid may be used to clean a tube or other element used in directing the jet of gas. For example, the system may be configured such that the buffer fluid drains directly into the tube or other element and washes away dust or other potential contaminants that may be present. A preliminary jet of gas may be blown through the tube (in a direction that is not towards the dispensing volume 322) to empty the tube or other element of the buffer fluid prior to applying a jet of gas to the dispensing volume 322 to deliver the dose. This preliminary jet of gas helps to ensure that the use of the buffer fluid for cleaning does not compromise the accuracy of the delivered dose. Blowing the buffer fluid vigorously out of the tube or other element may also dislodge further potential contaminants and generally assist with the cleaning process.

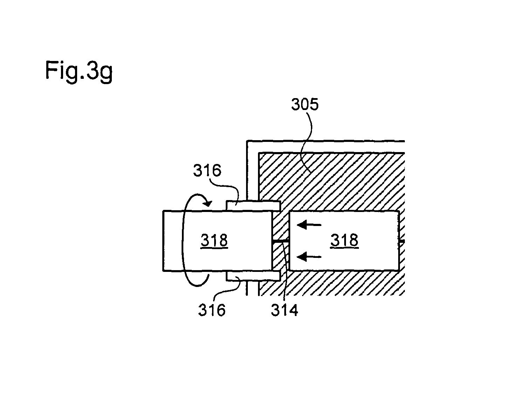

Figure 3g shows a dispenser in which the fluid to be dispensed is carried out of the reservoir 305 between segments 318 in the manner of the embodiment of Figures 3e and 3f, except that there is no provision for a buffer volume. It should be noted, however, that the principle of the embodiment of Figure 3g could also be applied in the case where a buffer volume is provided.

In the arrangement of Figure 3g, means are provided for controllably varying the dose to be presented at the dispensing point by varying the separation between segments 318 (and therefore the volume available for the fluid that is to be carried out of the reservoir 305 between the segments 318) within the reservoir 305 prior to their being pulled into the sliding seal 316. This variation could be achieved through a variety of mechanisms, but in the example shown the flexible connectors 314 separating the segments 318 are configured such that rotation of a segment 318 that is protruding out from the sliding 316 causes a changed in the axial separation between that segment 318 and an immediately preceding segment 318. This mechanism provides a convenient way of changing the amount of dose that will be delivered for a given actuation of the device (e.g. for a given jet of gas through the dispensing point).

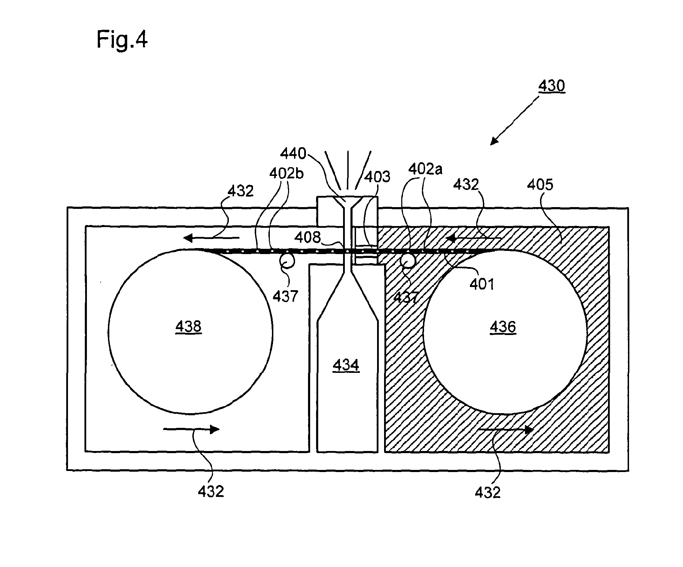

Figure 4 shows a side view of a treatment device 430 comprising a dispenser according to an embodiment of the invention. The transfer body 401 is of the flexible type, which can be formed into a coil, for example as shown in Figures 2a and 2b. To facilitate storage of the transfer body 401 and pulling of the transfer body 401 through the sliding seal 403, the transfer body may be wound onto spools 436 and 438 provided in the treatment device 430. For example, a supply spool 436 may be provided within the reservoir 405 and a take-up spool 438 may be provided in a region outside of the reservoir 405. Guide rollers 437 or the like may be provided to guide the transfer body 401 from the supply spool 436 through the sliding seal 403 to the take-up spool 438. Means may be provided for rotating the take-up spool 438 so as to unwind the transfer body from the supply spool 436 and pull it through the sliding seal and onto the take-up spool 438. In the particular example shown, such movement involves anti-clockwise rotation of the supply and guide spools (arrows 432).

As in previously described embodiments, cavities 402a/402b carry the fluid to be dispensed from the reservoir 405 through the sliding seal 403 to the dispensing point 408. In the present embodiment, the treatment device 430 comprises means 434 for blowing fluid contained in a cavity at the dispensing point 408 through an orifice 440 towards the site to which the fluid is to be applied (for example, onto the eye). However, many other techniques could be used for transferring liquid from the cavities to the site to be treated (or via which the treatment is to enter the body). For example, various means may be provided to dislodge the fluid from the cavities (either mechanically or manually): by flicking or shaking the transfer body, by applying vibrations, by using electrostatic attraction (or repulsion), or by using surface tension effects (for example, by bringing a surface for which the fluid has a high affinity into contact with the liquid held in the cavities).

The fluid could be dispensed, for example, as a single drop, in droplets, as a stream, or in a mist. The dispenser itself could be used in a variety of contexts. For example, the dispenser might be incorporated into a personal device to be used for regular treatment of the same individual. Alternatively, the device could be used in a hospital or pharmacy environment for dispensing individual doses of a medicament to various different people. The dispenser could form part of a surgical device.

The spool configuration illustrated is Figure 4 is merely exemplary and any one of a wide variety of mechanisms could be used for driving the transfer body through the sliding seal. Generally, such mechanisms need to be capable of driving the transfer body in a single direction only: once a portion of the transfer body has left the sliding seal and been exposed to the potentially non-sterile environment outside of the reservoir, it is undesirable for that portion of the transfer to re-enter the reservoir. Re-entry of portions of the transfer body containing or defining cavities could also introduce bubbles or other contaminants into the reservoir.

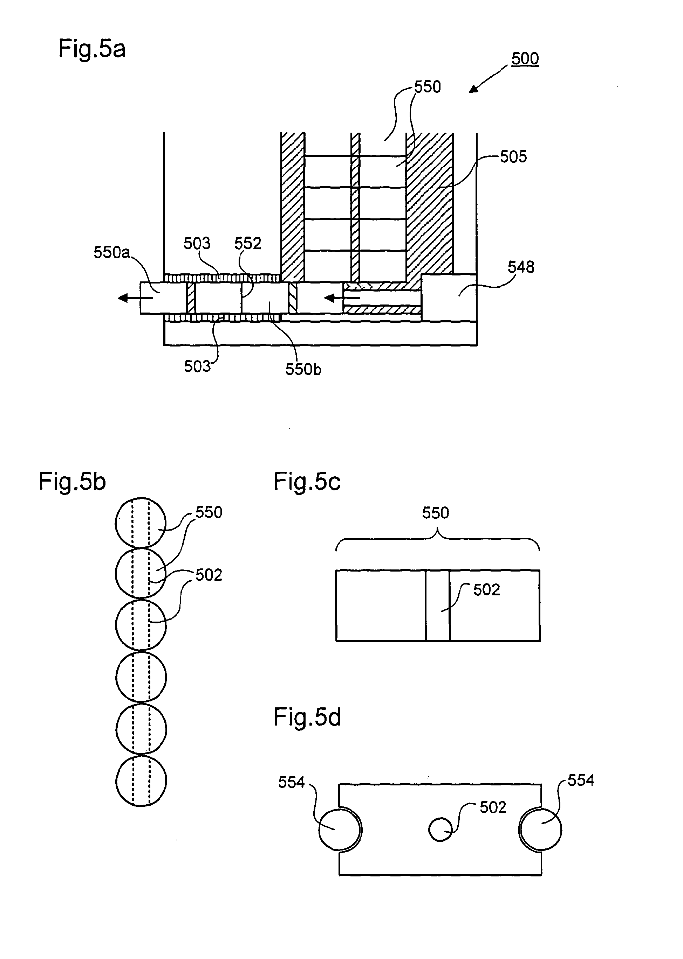

Figures 5a to 5d show a dispenser 500 according to an alternative embodiment of the invention. Figure 5a is a side view of the dispenser 500. Figure 5b is an end view of the transfer bodies 550 provided within the reservoir 505 of the dispenser 500 of Figure 5a. Figure 5c is a side view of one of the transfer bodies 550 in isolation. Figure 5d is a top view of the stack of transfer bodies 550 deployed within the reservoir 505 and aligned via alignment rods 554.

In the dispenser 500 of this embodiment, a plurality of disconnected transfer bodies 550 are provided, each having a single cavity 502 for storing and transporting the fluid to be dispensed. As can be seen in the side and end views of Figures 5b and 5c, each transfer body 550 has a cylindrical outer surface (not including the holes of the cavity 502), which will form the seal against the sliding seal 503 when the transfer body 550 is pushed through the sliding seal 503, and a single cylindrical cavity formed laterally through the transfer body 550 perpendicular to the axis of the cylindrical outer surface.

As shown in Figures 5b and 5d, the transfer bodies 550 are stacked on top of each other within the reservoir 505 and aligned with each other via vertical alignment rods 554.

The transfer bodies 550 are pushed through the sliding seal 503 by the pusher 548 located at the bottom of the stack. The system is configured so that the transfer bodies are pushed in series through the sliding seal 503, the pusher 548 applying a force to the transfer body 550a that is most advanced within the sliding seal 503 via the transfer body 550b that is immediately behind (i.e. via the interface 552 between the two transfer bodies 550a/b. In this way, it can be ensured that there is always at least one transfer body 550 within the seal 503 to maintain the integrity of the seal, even when one of the transfer bodies 550a is pushed completely out of the sliding seal 503.

The transfer bodies 550 are stacked vertically and aligned so that as the transfer bodies 550 are pushed progressively through the sliding seal 503, new transfer bodies 550 drop into position in front of the pusher 548 as they are needed. A receptacle (not shown) may be provided for receiving transfer bodies 550 after they have been used. For example, the system may be arranged such that the transfer bodies 550 are pushed into the receptacle after dispensing of their fluid by the motion of subsequent transfer bodies 550 and/or gravity.

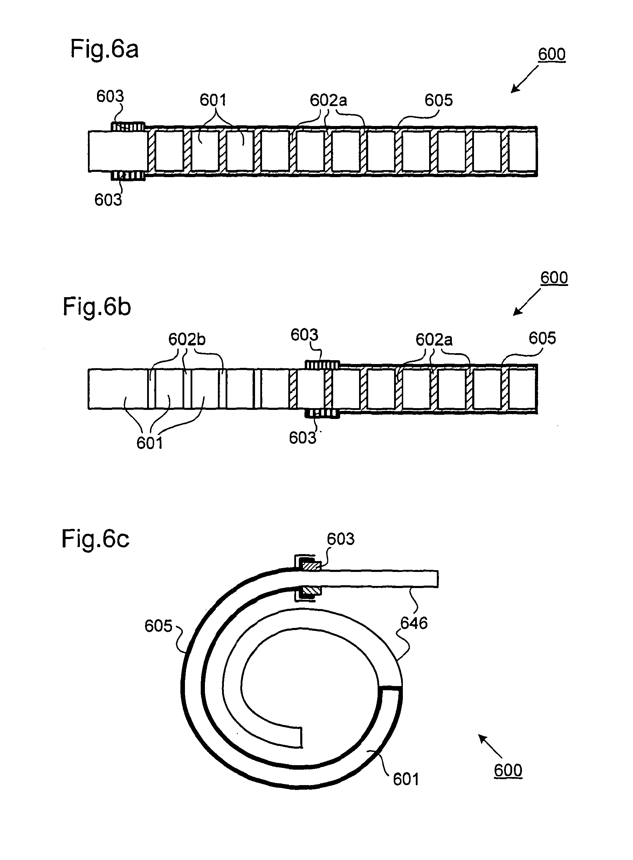

Figures 6a to 6c show a dispenser 600 according to an alternative embodiment of the invention. Figure 6a shows a side view of the dispenser 600 prior to dispensing of any fluid. Figure 6b shows a side view of the dispenser 600 after five doses of fluid have been passed through the sliding seal 603. Figure 6c shows a top view of the dispenser 600 when formed into a compact coil configuration and housed inside a reservoir casing tube 646.

In this embodiment, the reservoir 605 is formed from an elastic sleeve, which substantially conforms to the shape of the transfer body 601. The device is operated by moving (pulling, pushing, rotating, rolling or a combination thereof) the transfer body 601 through the sliding seal 603 in order to bring the fluid-containing cavities 602a from a position within the reservoir 605 to a dispensing point outside of the reservoir 605. A grip or connector portion (not shown) may be provided for a user to grip the device or as a connection point for installation in a treatment device comprising means to transfer fluid present in cavities at the dispensing point to the site to be treated. As successive cavities 602a are pushed through the sliding seal 603, the elastic sleeve of the reservoir 605 contracts so as to reduce the volume of the reservoir 605 as shown in Figure 6b. The transfer body 601 is shown in Figures 6a and 6b as having a straight, rod-like form, but other forms could also be used, including tape-like forms such as that shown in Figures 2a and 2b, as well as forms in which the transfer body 601 adopts a more compact configuration. In contrast to previously described embodiments in which such compact configurations are completed immersed within the fluid of the reservoir, the present embodiment allows for the possibility of the reservoir itself adopting the same compact configuration as the transfer body (because it can adopt the same shape as the transfer body). For example, the reservoir may adopt a coiled configuration as shown in Figure 6c. Such arrangements reduce the space required by the dispenser 600 and facilitate manufacture of compact treatment devices using the dispenser 600. Fluid waste is also minimized as a majority (or substantially all) of the fluid present is containing within the cavities 602a and can be dispensed.

Figure 6c shows the transfer body 601 and reservoir 605 in a coiled configuration. The reservoir 605 shortens as the transfer body is pushed past the sliding seal 603. A rigid reservoir casing tube 646 may be provided for protecting the reservoir 605 and/or transfer body 601 and/or for forcing the reservoir 605 and/or transfer body 601 to adopt the desired compact configuration (a coil in the embodiment shown), for example where the reservoir 605 and transfer body 601 are themselves flexible. As the transfer body 601 and reservoir advance, the rigid reservoir casing tube 646 is gradually emptied.

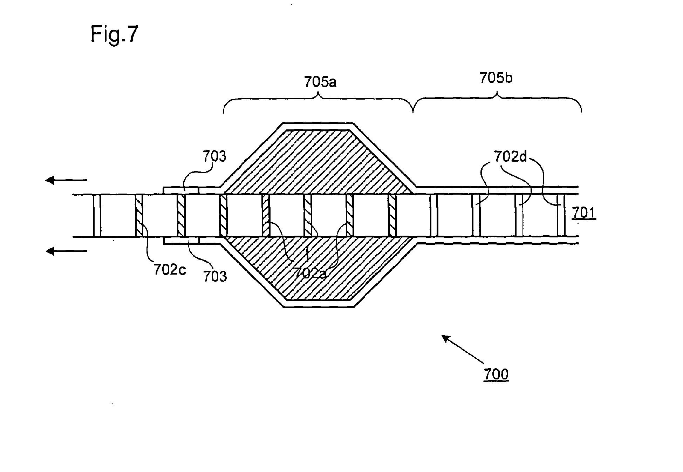

Figure 7 shows a dispenser 700 according to an alternative embodiment. Here, the transfer body 701 has a rod-like form similar to the transfer body 101 of the embodiment described above with reference to Figure 1. In contrast to the embodiment of Figure 1, however, where all of the cavities 102a that have not yet passed through the sliding seal 103 are filled with fluid (because they are all immersed within the reservoir), the reservoir 705 of the present embodiment is configured such that only a portion 702a of the cavities 702a/d that have not yet passed through the sliding seal 703 are filled with fluid. According to this embodiment, the reservoir has two sections: a fluid immersing section 705a and a sealing section 705b. The system is configured such that as the transfer body 701 is pushed progressively through the sliding seal 703, the cavities move from a position within the sealing section 705b (cavities 702d), where they are not in contact with fluid to be dispensed and are thus empty of fluid, through a range of positions within the fluid immersing section 705a (cavities 702a) where the cavities are immersed in the fluid to be dispensed and the cavities 702a are filled with fluid ready for passing through the sliding seal 703 to the dispensing point (cavities 702c). By only filling the cavities when needed, the size of the fluid containing part of the reservoir 705 can be made smaller, reducing the possibility of waste and facilitating compact manufacture.

In general, arrangements in which the reservoir contracts may advantageously be configured such that the contraction helps to ensure that the cavities leaving the reservoir are always completely full, so as to ensure accuracy of dose. Such arrangements may also be advantageous where the contraction of the reservoir is such as to compensate for the loss of volume of material within the reservoir caused by the transfer body being moved through the sliding seal (so as to avoid pressure drops within the reservoir which could inhibit movement of the transfer body and/or increase the risk of contaminants being sucked through the sliding seal).

In all of the embodiments discussed above, it is preferable to fill the cavities by completely immersing the cavities within the fluid to be dispensed and eliminating any gas from the reservoir.

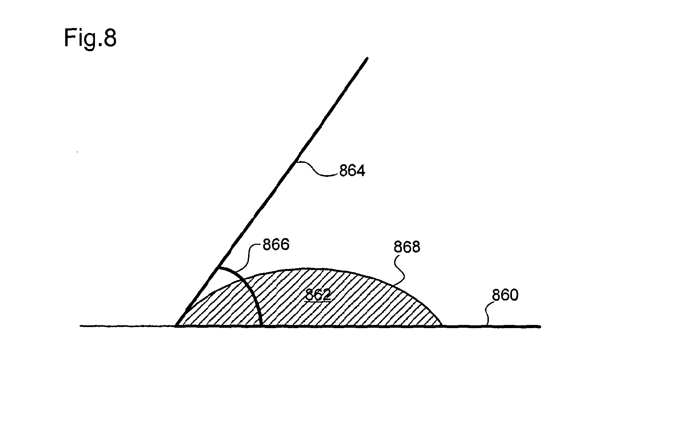

Figure 8 illustrates what is meant by "contact angle" (or, equivalently, "angle of contact") within the present application. The contact angle is a measure of the extent to which a fluid in contact with a particular solid surface "wets" that solid surface. Referring to Figure 8, the contact angle 866 is the angle between 1) the tangent 864 to the surface 868 of the droplet 862 of fluid at the point where the surface 868 of the droplet 862 comes into contact with the solid surface 860 and 2) the solid surface 860 underneath the droplet 862.

If there is a high energy associated with forming an interface between the fluid and the solid surface (relative to forming an air-solid interface and/or an air-fluid interface) then the fluid will tend to form a droplet with a high contact angle to minimize the size of the fluid-solid interface. On the other hand, if it is energetically more favourable to form a fluid-solid interface, then the fluid will tend to spread out more on the solid surface to increase the size of this interface, resulting in a lower contact angle.

The surface tension is a measure of the energy associated with the air-fluid interface; a high surface tension implies a low energy associated with the air-fluid interface. Thus, a high surface tension favours a high contact angle.

The contact angle can be adjusted by modifying the composition of the fluid. For example, the addition of inorganic salts to the fluid will tend to increase the surface tension and contact angle while the addition of alcohols or surfactants to the fluid will tend to decrease the surface tension and contact angle.

The contact angle can also be adjusted by modifying the composition of the solid surface, for example by forming the relevant structure (e.g. the transfer body) using a different material or by coating the relevant structure with a suitable material. Alternatively or additionally, the texture of the surface may be modified (which effectively changes the surface area). Alternatively or additionally, the solid surface may be chemically modified.

A surface which has a relatively low contact angle with water is commonly referred to as being "hydrophilic", while a surface which has a relatively high contact angle with water is commonly referred to as being hydrophobic.

Although the fluids to be used with the dispensers of the present invention will not in general be pure water they will frequently have an aqueous base and will typically behave in a similar way to water when brought into contact with surfaces of the dispenser. It is therefore useful to design the surface properties of the dispenser components with reference to water as a starting point. Where it is known what particular fluids are to be used, then the surface properties may be optimised accordingly if desired.

The contact angles between surfaces of the dispensers of the above-described embodiments can be modified to improve their performance in a number of ways.

For example, effective filling of the reservoirs can be aided by forming the reservoirs of (or coating their inside surfaces with) materials that have low contact angles with the fluid to be dispensed. This will help to avoid incomplete filling of the reservoirs as it will encourage effective wetting of all surfaces.

Effective holding of fluid doses in cavities in (or defined by segments of) the transfer bodies will be improved by arranging for the interior surfaces of the cavities to have a relatively low contact angle. Surfaces around the openings to these cavities may be arranged to have a much higher contact angle so as to prevent fluid escaping from (or being pulled out of) the cavities before dispensing occurs. On the other hand, the dispensing process itself may be hindered if the contact angle between the fluid and the cavities is too low, so that the choice of contact angle may preferably represent a balance between limiting leakage and promoting effective dispensing, at least in some applications.

More generally, any surfaces of the apparatus that may come into contact with the fluid to be dispensed, particularly where these surfaces are outside of the sliding seal, may beneficially be arranged to have a high contact angle with respect to the fluid to be dispensed so as to minimise the risk, or extent of, loss of fluid due to contact with these surfaces prior to dispensing of the fluid.

## Claims

1. A treatment device, comprising:
a fluid dispenser (100) comprising a transfer body (101); and
an applicator for applying a predetermined volume of fluid present at a dispensing point to a part of the human or animal body, further comprising:
a sealable reservoir (105) for storing a fluid to be dispensed;
wherein the transfer body (101) is configured to carry the predetermined volume of said fluid from said reservoir (105) to the dispensing point outside of said reservoir (105); and the dispenser **characterised in that** it further comprises:
a sliding seal (103) for maintaining said reservoir (105) in a sealed state while a portion of said transfer body (101) carrying said predetermined volume of fluid and said predetermined volume of fluid are moved from a position inside of said reservoir (105) to said dispensing point outside of said reservoir (105).

2. A treatment device according to claim 1, wherein said transfer body (101) is formed so to define a cavity (102a-c) capable of containing said predetermined volume of fluid, said transfer body (101) being arranged such that, in use, movement of a portion of said transfer body (101) defining said cavity (102a-c) from a position inside of said reservoir (105) to a position outside of said reservoir (105) causes said predetermined volume of fluid to be transferred from said reservoir (105) to said dispensing point outside of said reservoir (105), wherein, optionally:
an interior surface of said cavity (102a-c) is more hydrophilic than the other surfaces of said transfer body, optionally being formed such that a contact angle with water is less than 45 degrees;
a portion of the surface of said transfer body (101) surrounding an opening of said cavity is formed such that a contact angle with water is greater than 90 degrees;
a surface surrounding an outer opening of said sliding seal (103) is less hydrophilic than said interior surface of said cavity (102a-c);
said cavity (102a-c) has an opening into said reservoir (105) that is elongate in a direction perpendicular to a direction of insertion of said cavity (102a-c) through said sliding seal (103);
said cavity (102a-c) comprises two openings into said reservoir (105);
the device is configured such that when said cavity (102a-c) is at the dispensing point outside of said reservoir (105), said predetermined liquid is held against gravity in said cavity (102a-c) by surface tension; and/or
said reservoir is arranged such that fewer than all of the cavities (702a, 702d) in said transfer body (701) that are yet to be driven through said sliding seal (703) are filled with fluid.

3. A treatment device according to claim 2, wherein said transfer body (101) is formed so as to define a plurality of said cavities (102a-c), each capable of containing a predetermined volume of fluid, wherein, optionally:
said transfer body (101) is elongated, with a substantially constant cross-sectional profile and said sliding seal (103) is configured to have a cross-sectional profile substantially complimentary to that of said transfer body (101); and/or
said transfer body (101) is elongate and said plurality of cavities (102a-c) are spaced apart in a longitudinal direction of said transfer body.

4. A treatment device according to any one of the preceding claims, wherein said transfer body (301) is configured such that a portion of said transfer body (301) located inside of said reservoir can adopt a configuration that is more compact that the configuration adopted while that portion is moved through the sliding seal.

5. A treatment device according to claim 4, wherein each of said portions for carrying said predetermined volume of fluid from a position inside of said reservoir to a position outside of said reservoir is separated from a directly adjacent portion by a length of body (314) of lower rigidity, so as to facilitate adoption of said compact configuration, wherein, optionally:
each of said portions for carrying said predetermined volume of fluid and each of said lengths of body (314) of lower rigidity are formed from the same material, said lower rigidity being achieved by forming said lengths of body of lower rigidity so as to have a smaller cross-sectional area than that of each of the portions for carrying said predetermined volume of fluid, said transfer body (301) further comprising flexible filler members (312) formed between each of said portions for carrying said predetermined volume of fluid so that the transfer body (301) has a longitudinally constant cross-sectional profile, said flexible filler members (312) being formed from a material that is more flexible than that of said portions for carrying said predetermined volume of fluid and said lengths of body of lower rigidity (314); and/or
said compact configuration comprises one or more of the following: 1) a configuration in which said portions for carrying said predetermined volume of fluid form a coil; and 2) a configuration in which said bodies of lower rigidity are at least partially collapsed so as to allow the average separation between said portions for carrying said predetermined volume of fluid to be lower than is the case when said portions are being moved through the sliding seal and the bodies of lower rigidity are taut.

6. A treatment device according to any one of the preceding claims, wherein said transfer body (101) is a substantially cylindrical rod.

7. A treatment device according to any one of the preceding claims, wherein said fluid is for applying a treatment via the eye.

8. A treatment device according to any one of the preceding claims, wherein said predetermined volume of fluid is lower than 25 microlitres.

9. A treatment device according to any one of the preceding claims, wherein said reservoir (105) is configured to reduce in volume as the volume of fluid stored in said reservoir (105) is reduced, wherein, optionally, said reservoir is at least partially delimited by an elastic sleeve.

10. A treatment device according to any one of the preceding claims, wherein said portion of said transfer body (101) carrying said predetermined volume of fluid is completely submerged in said fluid when in said reservoir.

11. A treatment device according to any one of the preceding claims, wherein said transfer body (301) comprises a plurality of segments (318) having a cross-sectional profile that is such as to seal against said sliding seal (316), said segments (318) being connected to each other by flexible connectors (314), wherein said transfer body (301) is configured to carry said predetermined volume of fluid through said sliding seal (316) in a volume defined in a space between said segments (318).

12. A treatment device according to any of the preceding claims, wherein
said applicator comprises means for dispersing said predetermined volume of fluid in a jet of gas directed towards said part of the human or animal body.

13. A treatment device according to any one of the preceding claims, wherein said transfer body (101) is formed so as to define a plurality of said cavities (102a-c), each capable of containing a predetermined volume of fluid, and said treatment device is configured so that one or a plurality of different cavities (102a-c) can be selectively presented at said dispensing point at the same time and the fluid contained therein simultaneously applied, the volume of fluid applied depending on the number and nature of the cavities (102a-c) thus presented.

14. A method for dispensing a fluid, comprising:
sealing a fluid to be dispensed in a reservoir (105);
using a transfer body (101) to carry a predetermined volume of said fluid from said reservoir (105) to the dispensing point outside of said reservoir (105)
**characterised by** using a sliding seal (103) to maintain said reservoir (105) in a sealed state while a portion of said transfer body (101) carrying said predetermined volume of fluid and said predetermined volume of fluid are moved from a position inside of said reservoir (105) to said dispensing point outside of said reservoir (105).

15. A method according to claim 14, further comprising:
generating a jet of gas directed towards a part of a human or animal body to be treated; and
dispersing the predetermined volume of fluid present at said dispensing point in said jet of gas, wherein optionally the contact angles of said fluid relative to portions of the surfaces of said transfer body (101), said sliding seal (103) and/or said reservoir (105) are adjusted to aid filling of said reservoir (105) or dispensing of said fluid.

## Patentansprüche

1. Behandlungsvorrichtung umfassend:
einen Fluid-Spender (100), welcher einen Transportkörper (101) umfasst; und
einen Applikator, um ein vorbestimmtes Volumen eines Fluids, welches an einem Abgabepunkt vorhanden ist, auf einen Teil des menschlichen oder tierischen Körpers aufzubringen, darüber hinaus umfassend:
einen abdichtbaren Vorratsbehälter (105), um ein abzugebendes Fluid zu speichern;
wobei der Transportkörper (101) ausgestaltet ist, um das vorbestimmte Volumen des Fluids von dem Vorratsbehälter (105) zu dem Abgabepunkt außerhalb des Vorratsbehälters (105) zu befördern; und wobei der Spender **dadurch gekennzeichnet ist, dass** er darüber hinaus umfasst:
eine Gleitdichtung (103), um den Vorratsbehälter (105) in einem abgedichteten Zustand zu halten während ein Abschnitt des Transportkörpers (101), welcher das vorbestimmte Volumen des Fluids befördert, und das vorbestimmte Volumen des Fluids von einer Position innerhalb des Vorratsbehälters (105) zu dem Abgabepunkt außerhalb des Vorratsbehälters (105) bewegt werden.

2. Behandlungsvorrichtung nach Anspruch 1, wobei der Transportkörper (101) ausgebildet ist, um so einen Hohlraum (102a-c) zu definieren, welcher in der Lage ist, das vorbestimmte Volumen des Fluids zu enthalten, wobei der Transportkörper (101) ausgestaltet ist, so dass im Betrieb eine Bewegung eines Abschnitts des Transportkörpers (101), welcher den Hohlraum (102a-c) definiert, von einer Position innerhalb des Vorratsbehälters (105) zu einer Position außerhalb des Vorratsbehälters (105) bewirkt, dass das vorbestimmte Volumen des Fluids von dem Vorratsbehälter (105) zu dem Abgabepunkt außerhalb des Vorratsbehälters (105) transportiert wird, wobei optional:
eine innere Fläche des Hohlraums (102a-c) hydrophiler als die anderen Flächen des Transportkörpers ist, wobei sie optional ausgebildet ist, so dass ein Kontaktwinkel mit Wasser kleiner als 45 Grad ist;
ein Abschnitt der Oberfläche des Transportkörpers (101), welcher eine Öffnung des Hohlraums umgibt, ausgebildet ist, so dass ein Kontaktwinkel mit Wasser größer als 90 Grad ist;
eine Fläche, welche eine äußere Öffnung der Gleitdichtung (103) umgibt, weniger hydrophil ist, als die innere Fläche des Hohlraums (102a-c);
der Hohlraum (102a-c) eine Öffnung in dem Vorratsbehälter (105) aufweist, welche in eine Richtung senkrecht zu einer Richtung einer Einführung des Hohlraums (102a-c) durch die Gleitdichtung (103) länglich ist;
der Hohlraum (102a-c) zwei Öffnungen in dem Vorratsbehälter (105) umfasst;
die Vorrichtung ausgestaltet ist, so dass, wenn sich der Hohlraum (102a-c) an dem Abgabepunkt außerhalb des Vorratsbehälters (105) befindet, die vorbestimmte Flüssigkeit gegen die Schwerkraft durch Oberflächenspannung in dem Hohlraum (102a-c) gehalten wird; und/oder
der Vorratsbehälter angeordnet ist, so dass weniger als alle Hohlräume (702a, 702d) in dem Transportkörper (701), welche noch durch die Gleitdichtung (703) zu treiben sind, mit Fluid gefüllt sind.

3. Behandlungsvorrichtung nach Anspruch 2, wobei der Transportkörper (101) ausgebildet ist, um mehrere der Hohlräume (102a-c) zu definieren, wobei jeder in der Lage ist, ein vorbestimmtes Volumen des Fluids zu enthalten, wobei optional:
der Transportkörper (101) länglich mit einem im Wesentlichen konstanten Querschnittsprofil ist, und wobei die Gleitdichtung (103) ausgestaltet ist, um ein Querschnittsprofil aufzuweisen, welches im Wesentlichen komplementär zu demjenigen des Transportkörpers (101) ist; und/oder
der Transportkörper (101) länglich ist und die mehreren Hohlräume (102a-c) in einer Längsrichtung des Transportkörpers beabstandet sind.

4. Behandlungsvorrichtung nach einem der vorhergehenden Ansprüche, wobei der Transportkörper (301) ausgestaltet ist, so dass ein Abschnitt des Transportkörpers (301), welcher innerhalb des Vorratsbehälters angeordnet ist, eine Konfiguration annehmen kann, welche kompakter als die Konfiguration ist, welche angenommen wird, während der Abschnitt durch die Gleitdichtung bewegt wird.

5. Behandlungsvorrichtung nach Anspruch 4, wobei jeder der Abschnitte zum Befördern des vorbestimmten Volumens des Fluids von einer Position innerhalb des Vorratsbehälters zu einer Position außerhalb des Vorratsbehälters von einem direkt benachbarten Abschnitt um eine Länge eines Körpers (314) einer geringeren Steifigkeit abgetrennt ist, um so das Annehmen der kompakten Konfiguration zu ermöglichen, wobei optional:
jeder der Abschnitte zum Befördern des vorbestimmten Volumens des Fluids und jeder der Längen des Körpers (314) einer geringeren Steifigkeit aus demselben Material ausgebildet sind, wobei die geringere Steifigkeit erzielt wird, indem die Längen des Körpers der geringeren Steifigkeit ausgebildet werden, um einen kleineren Querschnittsbereich als den von jedem der Abschnitte zum Befördern des vorbestimmten Volumens des Fluids aufzuweisen, wobei der Transportkörper (301) darüber hinaus flexible Füllteile (312) umfasst, welche zwischen den jeweiligen Abschnitten zum Befördern des vorbestimmten Volumens des Fluids ausgebildet sind, so dass der Transportkörper (101) ein in Längsrichtung konstantes Querschnittsprofil aufweist, wobei die flexiblen Füllteile (312) aus einem Material ausgebildet sind, welches flexibler als dasjenige der Abschnitte zum Befördern des vorbestimmten Volumens des Fluids und der Längen des Körpers der geringeren Steifigkeit (114) ist; und/oder
die kompakte Konfiguration eine oder mehrere der folgenden umfasst: 1) eine Konfiguration, in welcher die Abschnitte zum Befördern des vorbestimmten Volumens des Fluids eine Spirale ausbilden; und 2) eine Konfiguration, in welcher die Körper der geringeren Festigkeit zumindest teilweise zusammengelegt sind, um so zu ermöglichen, dass der mittlere Abstand zwischen den Abschnitten zum Befördern des vorbestimmten Volumens des Fluids geringer als in dem Fall ist, wenn die Abschnitte durch die Gleitdichtung bewegt werden und die Körper der geringeren Steifigkeit straff sind.

6. Behandlungsvorrichtung nach einem der vorhergehenden Ansprüche, wobei der Transportkörper (101) ein im Wesentlichen zylindrischer Stab ist.

7. Behandlungskosten nach einem der vorhergehenden Ansprüche, wobei das Fluid zum Anwenden einer Behandlung über das Auge ist.

8. Behandlungsvorrichtung nach einem der vorhergehenden Ansprüche, wobei das vorbestimmte Volumen des Fluids kleiner als 25 Mikroliter ist.

9. Behandlungsvorrichtung nach einem der vorhergehenden Ansprüche, wobei der Vorratsbehälter (105) ausgestaltet ist, um sich bezüglich des Volumens zu verringern, wenn sich das Volumen des Fluids, welches in dem Vorratsbehälter (105) gespeichert ist, verringert, wobei der Vorratsbehälter zumindest teilweise durch eine elastische Hülle abgegrenzt ist.

10. Behandlungsvorrichtung nach einem der vorhergehenden Ansprüche, wobei der Abschnitt des Transportkörpers (101), welcher das vorbestimmte Volumen des Fluids befördert, vollständig in dem Fluid untergetaucht ist, wenn er sich in dem Vorratsbehälter befindet.

11. Behandlungsvorrichtung nach einem der vorhergehenden Ansprüche, wobei der Transportkörper (301) mehrere Segmente (318) umfasst, welche ein Querschnittsprofil aufweisen, welches vorhanden ist, um gegen die Gleitdichtung (316) abzudichten, wobei die Segmente (318) durch flexible Verbindungselemente (314) miteinander verbunden sind, wobei der Transportkörper (301) ausgestaltet ist, um das vorbestimmte Volumen des Fluids durch die Gleitdichtung (316) in einem Volumen zu befördern, welches in einem Zwischenraum zwischen den Segmenten (318) definiert ist.

12. Behandlungsvorrichtung nach einem der vorhergehenden Ansprüche, wobei der Applikator Mittel umfasst, um das vorbestimmte Volumen des Fluids in einem Gasstrom, welcher zu dem Teil des menschlichen oder tierischen Körpers gerichtet ist, abzugeben.

13. Behandlungsvorrichtung nach einem der vorhergehenden Ansprüche, wobei der Transportkörper (101) ausgebildet ist, um so mehrere der Hohlräume (102a-c) zu definieren, wobei jeder in der Lage ist, ein vorbestimmtes Volumen des Fluids zu enthalten, und wobei die Behandlungsvorrichtung ausgestaltet ist, so dass ein oder mehrere der verschiedenen Hohlräume (102a-c) selektiv an dem Abgabepunkt zur selben Zeit vorhanden sein können und das Fluid, welches darin enthalten ist, gleichzeitig aufgebracht wird, wobei das Volumen des Fluids, welches aufgebracht wird, von der Anzahl und der Eigenschaft der Hohlräume (102a-c), welche dadurch vorhanden sind, abhängt.

14. Verfahren zum Abgeben eines Fluids, umfassend:
Abdichten eines abzugebenden Fluids in einem Vorratsbehälter (105);
Einsetzen eines Transportkörpers (101), um ein vorbestimmtes Volumen des Fluids von dem Vorratsbehälter (105) zu dem Abgabepunkt außerhalb des Vorratsbehälters (105) zu befördern,
**gekennzeichnet durch**
Einsetzen einer Gleitdichtung (103), um den Vorratsbehälter (105) in einem abgedichteten Zustand zu halten während ein Abschnitt des Transportkörpers (101), welcher das vorbestimmte Volumen des Fluids befördert, und das vorbestimmte Volumen des Fluids von einer Position innerhalb des Vorratsbehälters (105) zu dem Abgabepunkt außerhalb des Vorratsbehälters (105) bewegt werden.

15. Verfahren nach Anspruch 14, darüber hinaus umfassend:
Erzeugen eines Gasstroms, welcher zu einem Teil eines menschlichen oder tierischen Körpers, welcher zu behandeln ist, gerichtet ist; und
Abgeben des vorbestimmten Volumens des Fluids, welches an dem Abgabepunkt vorhanden ist, in den Luftstrom, wobei optional die Kontaktwinkel des Fluids relativ zu Abschnitten der Flächen des Transportkörpers (101), der Gleitdichtung (103) und/oder des Vorratsbehälters (105) eingestellt werden, um ein Befüllen des Vorratsbehälters (105) oder ein Abgeben des Fluids zu unterstützen.

## Revendications

1. Dispositif de traitement, comportant :
un distributeur (100) de fluide comprenant un corps de transfert (101) ; et
un applicateur pour appliquer un volume prédéterminé de fluide, présent en un point de distribution, sur une partie du corps humain ou animal, comportant en outre :
un réservoir (105) à fermeture hermétique pour stocker un fluide à distribuer ;
le corps de transfert (101) étant conçu pour acheminer le volume prédéterminé dudit fluide dudit réservoir (105) au point de distribution à l'extérieur dudit réservoir (105) ; et le distributeur étant **caractérisé en ce qu'**il comprend en outre :
un joint glissant (103) pour maintenir hermétiquement fermé ledit réservoir (105) pendant qu'une partie dudit corps de transfert (101) pour l'acheminement dudit volume prédéterminé de fluide et ledit volume prédéterminé de fluide passent d'une position à l'intérieur dudit réservoir (105) audit point de distribution à l'extérieur dudit réservoir (105).

2. Dispositif de traitement selon la revendication 1, dans lequel ledit corps de transfert (101) est formé de manière à définir une cavité (102a-c) apte à contenir ledit volume prédéterminé de fluide, ledit corps de transfert (101) étant agencé de telle sorte que, en service, le mouvement d'une partie dudit corps de transfert (101) définissant ladite cavité (102a-c) depuis une position à l'intérieur dudit réservoir (105) jusqu'à une position à l'extérieur dudit réservoir (105) amène ledit volume prédéterminé de fluide à être transféré dudit réservoir (105) audit point de distribution à l'extérieur dudit réservoir (105), dans lequel, éventuellement :
une surface intérieure de ladite cavité (102a-c) est plus hydrophile que les autres surfaces dudit corps de transfert, étant éventuellement formée de façon qu'un angle de contact avec l'eau soit inférieur à 45 degrés ;
une partie de la surface dudit corps de transfert (101) entourant une ouverture de ladite cavité est formée de façon qu'un angle de contact avec l'eau soit supérieur à 90 degrés ;
une surface entourant une ouverture extérieure dudit joint glissant (103) est moins hydrophile que ladite surface intérieure de ladite cavité (102a-c) ;
ladite cavité (102a-c) a une ouverture débouchant dans ledit réservoir (105) qui est allongée dans une direction perpendiculaire à une direction d'insertion de ladite cavité (102a-c) à travers ledit joint glissant (103) ;
ladite cavité (102a-c) comprend deux ouvertures débouchant dans ledit réservoir (105) ;
le dispositif est conçu de telle sorte que, lorsque ladite cavité (102a-c) est au point de distribution à l'extérieur dudit réservoir (105), ledit liquide prédéterminé soit maintenu, par tension de surface, en opposition à la pesanteur dans ladite cavité (102a-c) ; et/ou
ledit réservoir est agencé de façon que moins que la totalité des cavités (702a, 702d) dans ledit corps de transfert (701) qui restent à faire passer à travers ledit joint glissant (703) soient remplies de fluide.

3. Dispositif de traitement selon la revendication 2, dans lequel ledit corps de transfert (101) est formé de manière à définir une pluralité desdites cavités (102a-c), aptes à contenir chacune un volume prédéterminé de fluide, dans lequel, éventuellement :
ledit corps de transfert (101) est allongé, avec un profil en coupe sensiblement constant et ledit joint glissant (103) est conçu de façon à avoir un profil en coupe sensiblement complémentaire de celui dudit corps de transfert (101) ; et/ou
ledit corps de transfert (101) est allongé et ladite pluralité de cavités (102a-c) sont espacées les unes des autres dans une direction longitudinale dudit corps de transfert.

4. Dispositif de traitement selon l'une quelconque des revendications précédentes, dans lequel ledit corps de transfert (301) est conçu de façon qu'une partie dudit corps de transfert (301) située à l'intérieur dudit réservoir puisse adopter une configuration plus compacte que la configuration adoptée pendant que cette partie est amenée à passer à travers le joint glissant.

5. Dispositif de traitement selon la revendication 4, dans lequel chacune desdites parties pour l'acheminement dudit volume prédéterminé de fluide depuis une position à l'intérieur dudit réservoir jusqu'à une position à l'extérieur dudit réservoir est séparée d'une partie directement adjacente par une longueur de corps (314) moins rigide de façon à faciliter l'adoption de ladite configuration compacte, dans lequel, éventuellement :
lesdites parties pour l'acheminement dudit volume prédéterminé de fluide et chacune desdites longueurs de corps (314) moins rigides sont faites chacune de la même matière, ladite moindre rigidité étant obtenue en formant lesdites longueurs de corps moins rigides de telle sorte qu'elles aient une section transversale plus petite que celle de chacune des parties pour l'acheminement dudit volume prédéterminé de fluide, ledit corps de transfert (301) comprenant en outre des éléments de remplissage souples (312) formés entre chacune desdites parties pour l'acheminement dudit volume prédéterminé de fluide de façon que le corps de transfert (301) ait un profil en coupe sensiblement constant, lesdites éléments de remplissage souples (312) étant faits d'une matière plus souple que celle desdites parties pour l'acheminement dudit volume prédéterminé de fluide et desdites longueurs de corps moins rigides (314) ; et/ou
ladite configuration compacte comprend l'une et/ou l'autre des configurations suivantes : 1) une configuration dans laquelle lesdites parties pour l'acheminement dudit volume prédéterminé de fluide forment un serpentin ; et 2) une configuration dans laquelle lesdits corps moins rigides sont au moins partiellement écrasés afin de permettre que la séparation moyenne entre lesdites parties pour l'acheminement dudit volume prédéterminé de fluide soit moindre que dans le cas où lesdites parties sont en train de passer à travers le joint glissant et les corps moins rigides sont tendus.

6. Dispositif de traitement selon l'une quelconque des revendications précédentes, dans lequel ledit corps de transfert (101) est une tige sensiblement cylindrique.

7. Dispositif de traitement selon l'une quelconque des revendications précédentes, dans lequel ledit fluide sert à appliquer un traitement par l'oeil.

8. Dispositif de traitement selon l'une quelconque des revendications précédentes, dans lequel ledit volume prédéterminé de fluide est inférieur à 25 microlitres.

9. Dispositif de traitement selon l'une quelconque des revendications précédentes, dans lequel le volume dudit réservoir (105) est conçu pour diminuer à mesure que diminue le volume de fluide stocké dans ledit réservoir (105), dans lequel, éventuellement, ledit réservoir est au moins partiellement délimité par un manchon élastique.

10. Dispositif de traitement selon l'une quelconque des revendications précédentes, dans lequel ladite partie dudit corps de transfert (101) pour l'acheminement dudit volume prédéterminé de fluide est complètement plongée dans ledit fluide quand elle est dans le réservoir.

11. Dispositif de traitement selon l'une quelconque des revendications précédentes, dans lequel ledit corps de transfert (301) comprend une pluralité de segments (318) à profil en coupe tel qu'une étanchéité est obtenue contre ledit joint glissant (316), lesdits segments (318) étant reliés les uns aux autres par des raccords souples (314), ledit corps de transfert (301) étant conçu pour acheminer ledit volume prédéterminé de fluide à travers ledit joint glissant (316) en un volume défini dans un espace entre lesdits segments (318).

12. Dispositif de traitement selon l'une quelconque des revendications précédentes, dans lequel
ledit applicateur comprend un moyen pour disperser ledit volume prédéterminé de fluide dans un jet de gaz dirigé vers ladite partie du corps humain ou animal.

13. Dispositif de traitement selon l'une quelconque des revendications précédentes, dans lequel ledit corps de transfert (101) est formé de manière à définir une pluralité desdites cavités (102a-c) aptes à contenir chacune un volume prédéterminé de fluide, et ledit dispositif de traitement est conçu de telle sorte qu'une seule ou une pluralité de cavités différentes (102a-c) puisse(nt) être présentée(s) sélectivement et en même temps audit point de distribution et que le fluide contenu dans celle(s)-ci soit simultanément appliqué, le volume de fluide appliqué dépendant du nombre et de la nature des cavités(102a-c) ainsi présentées.

14. Procédé pour distribuer un fluide, comportant :
l'enfermement hermétique, dans un réservoir (105), d'un fluide à distribuer ;
l'utilisation d'un corps de transfert (101) pour acheminer un volume prédéterminé dudit fluide dudit réservoir (105) au point de distribution à l'extérieur dudit réservoir (105) ;
**caractérisé par**
l'utilisation d'un joint glissant (103) pour maintenir ledit réservoir (105) dans un état hermétiquement fermé pendant qu'une partie dudit corps de transfert (101) pour l'acheminement dudit volume prédéterminé de fluide et ledit volume prédéterminé de fluide sont amenés à passer d'une position à l'intérieur dudit réservoir (105) audit point de distribution à l'extérieur dudit réservoir (105).

15. Procédé selon la revendication 14, comportant en outre :
la création d'un jet de gaz dirigé vers une partie d'un corps humain ou animal à traiter ; et
la dispersion du volume prédéterminé de fluide présent audit point de distribution dans ledit jet de gaz, dans lequel, éventuellement, les angles de contact dudit fluide par rapport à des parties des surfaces dudit corps de transfert (101), dudit joint glissant (103) et/ou dudit réservoir (105) sont réglés pour faciliter le remplissage dudit réservoir (105) ou la distribution dudit fluide.
